Europäisches Patentamt

European Patent Office

Office européen des brevets

(11) Publication number: **0 270 120**
**A2**

# EUROPEAN PATENT APPLICATION

(21) Application number: 87117911.5

(22) Date of filing: 03.12.87

(51) Int. Cl.⁴: **A01G 7/00** , C12N 15/00 ,
C12N 5/00 , //A01H1/00

(30) Priority: 04.12.86 US 937724

(43) Date of publication of application:
08.06.88 Bulletin 88/23

(84) Designated Contracting States:
AT BE CH DE ES FR GB GR IT LI LU NL SE

(71) Applicant: DNA PLANT TECHNOLOGY
CORPORATION (under the laws of the state
of Delaware)
2611 Branch Pike
Cinnaminson, New Jersey(US)

(72) Inventor: Evans, David Allen
917 Lincoln Avenue
Palmyra New Jersey 08065(US)

(74) Representative: Patentanwälte Grünecker,
Kinkeldey, Stockmair & Partner
Maximilianstrasse 58
D-8000 München 22(DE)

(54) A process for breaking genetic linkage.

(57) A method for breaking genetic linkage useful for separating desirable traits from undesirable traits in hybrid plants comprising:

a) culturing a callus from a explant of a hybrid plant or from a somatic cell hybrid on a basal medium containing a somatic recombination inducing amount of growth regulator substances and regenerating shoots from said callus;

b) rooting said regenerated shoots on a rooting medium to form regenerated plantlets;

c) growing said plantlets into mature plants; and

d) recovering those plants displaying a recombinant phenotype.

Figure 1

EP 0 270 120 A2

## A PROCESS FOR BREAKING GENETIC LINKAGE

### Field of the Invention

This invention relates to a process for breaking genetic linkage in tissue culture regenerable species of plants. More specifically this invention relates to a process for breaking genetic linkage in tomato.

### Background of the Invention

The fundamental nature of the transmission of genetic information from generation to generation is generally well understood. The information is encoded into discrete units called genes. Chemically a gene is a polymer of deoxyribonucleic acid (DNA) and conventionally exists as a double-stranded polymer of helical configuration. The chromosome represents the means by which large numbers of genes are organized to insure efficient replication and transmission to daughter cells during cell division. Chromosomes are comprised of a single duplex DNA polymer of great length. Thus the information encoded by a large number of genes is contained on a single chromosome. Also existing as part of the chromosome are structural components in the form of proteins. In a fanciful analogy the organization of genetic information was viewed as being "beads on a string" with the beads representing individual genes and the string the chromosome. Although this simplification was shown not to be accurate, more recent electron micrographic studies of the macromolecular complexes of chromosomal DNA and associated protein revealed a repeating spheroid chromatin unit called a nu body, thus the analogy may have been in part prophetic.

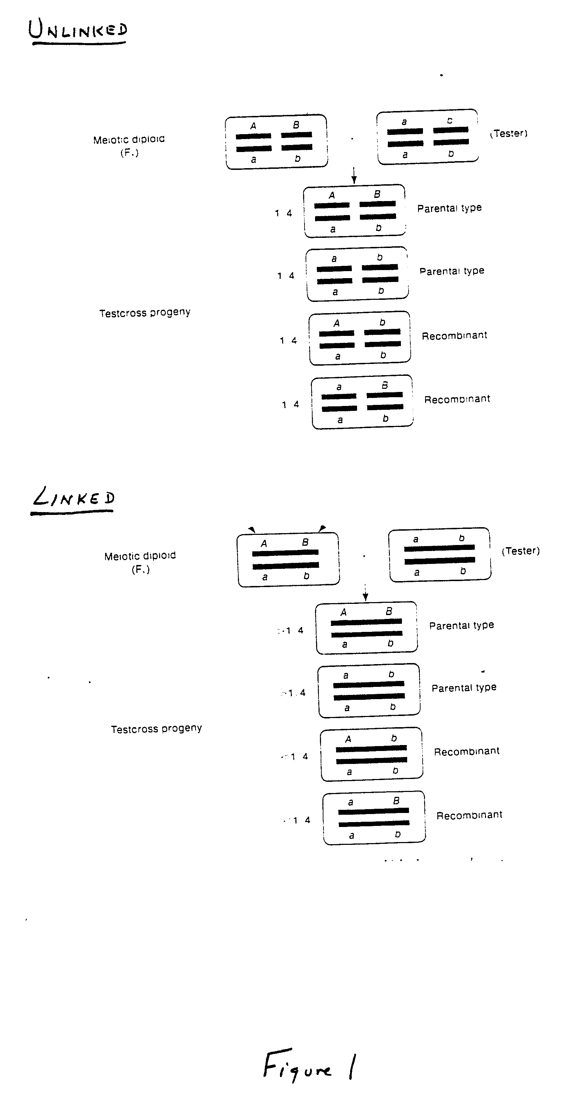

When two different genes reside on the same chromosome they are said to be linked, whereas if the two genes reside on different chromosomes, the genes are said to be unlinked. The prediction of the behavior of unlinked genes is provided by Mendel's second law, different segregating genes behave independently (i.e. the law of independent assortment). When genes are linked their assortment is not independent rather it is dependent. These two types of behavior are illustrated in Figure 1 by employing the genetic analytical tool of a test cross. It can be seen that even though genes are linked, that some degree of recombination is possible. The frequency of recombination is to at least a first approximation a function of the physical location of the genes. It is now appreciated that the molecular process responsible for generating the intrachromosomal recombinants involves the physical breaking and rejoining of chromosome segments, the so-called cross-over event. If the probability of a cross-over event occurring is random along the length of a chromosome, then the closer the two genes are, the less likely it is that a cross-over event will recombine the alleles of the two genes. Since the recombination frequencies between a number of genes have been experimentally determined it is possible to construct a genetic map which gives the relative location of each gene based on its frequency of recombination with other genes. It should be remembered that such genetic maps (or linkage maps) are statistical constructions based upon the analysis of the number of recombinant phenotypes generated in test cross experiments. There is a strong implication that the distance between gene pairs on a linkage map represent the physical distance along a chromosome, however as discussed below such may not always be the case.

The preceding discussion was predicated on the assumption that cross-over events were random occurrences along the entire length of the chromosome, however, for not particularly well understood physical and molecular reasons, cross-overs are at times not random occurrences. For example, the frequency of cross-overs seems to be suppressed when genes lie near centromeres. Centromeres being the location on a chromosome where spindle fibers attach facilitating chromosome migration during cell division. Further the presence of a first cross-over at a region of a chromosome can either enhance (statistically increase) the likelihood of a second cross-over occurring nearby or interfere (statistically decrease) the likelihood a second nearby cross-over.

In the course of crop improvement projects involving selective breeding protocols, it is often desirable to disrupt the linkage of a first gene encoding a desirable trait and a nearby gene encoding a less desirable or even detrimental trait. Reliance upon meiotic recombination to generate the desirable recombinants is unsuitable if the two genes are tightly linked.

This invention provides a means for generating recombination between tightly linked genes and a method for recovering the recalcitrant recombinant phenotypes resulting therefrom.

## Brief Description of the Invention

This invention provides a method for breaking genetic linkage useful for separating desirable traits from undesirable traits in hybrid plants comprising:

a) culturing a callus from a explant of a hybrid plant or from a somatic cell hybrid on a basal medium containing a somatic recombination inducing amount of growth regulator substances and regenerating shoots from said callus;

b) rooting said regenerated shoots on a rooting medium to form regenerated plantlets;

c) growing said plantlets into mature plants; and

d) recovering those plants displaying a recombinant phenotype.

This invention also provides a method of introgressing useful traits from a wild species into a cultivated species comprising:

a) forming a hybrid between a wild species possessing desirable and undesirable trait and a cultivated variety;

b) culturing a callus from an explant of said hybrid on a basal medium containing a somatic recombination inducing amount of growth regulator substances and regenerating shoots from said callus;

c) rooting said regenerated shoots on a rooting medium to form regenerated plantlets;

d) growing said plantlets into mature ($R_o$) plants; and

e) recovering the plants which display the desired trait in combination with the traits associated with the cultivated variety.

## Detailed Description of the Figures

Figure 1 illustrates the results of a test cross experiment when genes are unlinked and when genes are linked.

Figure 2 illustrates mitotic cross-over events which yield various phenotypes in tissue culture regenerated progeny.

Figure 3 illustrates a partial linkage map of chromosome 6 in tomato.

Figure 4 illustrates somatic recombinants recovered displaying a variety of leaf shape morphology.

Figure 5 illustrates somatic recombinants for flower shape.

## Detailed Description of the Invention

The foregoing discussion regarding the nature of gene linkage and the ease or the difficulty with which the linkage may be broken was predicated on the results of experiments involving meiotic cells and the scoring of progeny arising from recombinant gametes after fertilization. Meiotic recombination is defined as any meiotic process that generates a haploid product of meiosis the genotype of which is different from the two haploid genotypes that constituted the meiotic diploid. The product of meiosis so generated is called a recombinant. It has been appreciated that recombination and segregation can also occur in mitotic cells. Mitotic recombination must be studied in diploid cells and may be defined as any mitotic process that generates a diploid daughter cell with a combination of genes different from the diploid parental cell in which the mitosis occurred. Mitotic events associated with such results include mitotic nondisjunction, mitotic chromosome loss and mitotic crossing-over.

The effect of a mitotic recombinational event in a multicellular organism is to generate a section of tissue composed of cells of recombinant genotype, often recognizable because of their different phenotype compared to that of parental tissue which surrounds the cells. The term mosaic is used to describe this phenomenon. In the cases of mitotic crossing-over, since the event is reciprocal, twin spots can occur. Twin spots being two adjacent areas of recombinant genotypes yielding different phenotypes. Such twinning has been studied in Drosophilia (Stern, C., Genetics 21: 625-730 (1936)) and in plants (Evans, D.A., and E.F. Paddock, In: Plant Cell and Tissue Culture: Principles and Applications, W.R. Sharp et al., Eds., Ohio State Univ Press, 1979, pp. 315-351).

Mitotic crossing-over has been suggested as one source of variation observed in tissue culture regenerated plants (D.A. Evans and W.R. Sharp, Science 221:949-951 (1983); and D.A. Evans, W.R. Sharp and H.P. Medine-Filho, Amer. Jour. Bot. 71:759-774 (1984)), although the genetic proof of such a mechanism is lacking. This invention provides a method for inducing mitotic crossing over in tissue culture and for recovering recombinant plants produced thereby.

By way of examplification, the invention is demonstrated by breaking the linkage of two genes in tomato. The fact that the tomato system is used to illustrate the invention should not limit the invention. The invention is applicable to any plant species which is tissue culture regenerable.

In tomatoes, nematode infestation is manifested by the development of shallow and knotted root systems. The infected plants wilt despite adequate irrigation and suffer from mineral deficiency, leading to reduced top growth and decreased fruit yield. Two species of root knot nematodes, Meloidogyne incognita and M. javanica, are prevalent in the Sacramento and San Joaquin Valleys where most processing tomatoes are grown. Chemical control is costly and has limited efficacy. Instead, efforts to transfer natural resistance from wild tomato species have been far more successful.

Nematode resistance was originally introduced into Lycopersicon esculentum via a cross with L. peruvianum var. dentatum. The trait has been characterized as a single dominant gene (Mi) at position 35 cM on chromosome 6 and confers resistance to M. incognita , M. javanica, M. acrita, and M. arenariaia. Rick and Fobes (Tomato Genet. Coop. Rep. 24:25 (1974)) subsequently observed that the Mi locus was tightly linked to an isozyme marker, acid phosphatase 1 (Aps-1). Thus, nematode resistance can be determined by staining for Aps-1. Plants which synthesize the fast migrating Aps-1 polypeptide, otherwise restricted to accessions of L. peruvianum var. dentatum, have functional nematode resistance.

While the incorporation of two copies of the resistance allele would greatly simplify commercial tomato breeding schemes, this gene combination also results in soft fruit with high stem retension and reduced yield. Considering the source of the resistance, such effects have been attributed to tightly linked deleterious gene combinations rather than a pleiotropic effect of the Mi locus. Extensive efforts to break this linkage have not been successful, possibly due to the proximity of Mi to the centromere, where meiotic recombination is suppressed.

MCO stock, heterozygous for the following traits, yv, Mi, Aps-1, coa, c, were obtained from Dr H.P. Medina-Filho, Instituto Agronomico, Sao Paulo, Brazil. A brief descripton of each trait, all located on the long arm of chromosome 6, is provided in Table 1.

## TABLE 1

### SUMMARY OF GENETIC MARKERS ON CHROMOSOME 6

| Symbol | Description of trait | Chromosome position |
|---|---|---|
| yv | yellow virescence | 34 |
| Mi | nematode resistance | 35 |
| Aps-1 | acid phosphatase | 35 |
| coa | corrotundata | 64 |
| c | potato leaf | 93 |

Young leaf tissues were placed into culture according to the following procedure.

The process is initiated by placing into tissue culture, an explant of tissue from the plant variety in which new variation is desired. The plant tissue is preferably sterilized prior to growing the callus. This prevents microorganisms which grow faster than the plant tissue from contaminating the culture. Methods of sterilization are known in the art. A simple and economical method is to immerse the tissue in a 7% solution of household chlorine bleach (e.g., Clorox) for about 10 minutes and then rinse the tissue two or three times with sterile distilled water. A wide variety of tissue sources are known. Explants of cotyledons, hypocotyl, stem, leaf, shoot apex, root, young inflorescenes, flower petals, petioles, ovular tissues and embryos are useful sources according to the instant invention. The present invention accomplishes the mitotic recombination generating step by employing growth regulators at unusually high concentration in the basal tissue culture medium. Useful growth regulators in the context of the subject invention comprise cytokinins such as 6-benzyladenine (6-BA), kinetin, zeatin, and 2-isopentyladenine (2-iP) and auxins such as indole acetic acid (IAA), indole butyric acid (IBA), napthalene acetic acid (NAA), and 2,4-dichlorophenoxyacetic acid (2,4-D). Sterile plant tissue is then transferred to a basal medium containing 6-BA. Basal media comprise inorganic nutrients, vitamins and a carbon source. The selection of an appropriate solid basal media are known, including but not limited to, B5 medium, White's medium and Schenk-Hildebrandt medium. See, e.g., Gamborg, et al., (1976), In Vitro 12: 473-478. The preferred medium is that described by Murashige and Skoog, (1962), Physiol. Plant. 15: 473-497 (MS medium).

The present invention requires that 6-BA be present in the medium at a concentration of at least about 5 uM, preferably at least about 10 uM, to obtain the desired results. As noted above, the number of variant plants obtained is related to the concentration of the 6-BA. An increase in the number of variants has been observed as the 6-BA concentration was increased. It may be desirable, therefore, to increase the concentration of the 6-BA above 5 or 10 uM level. It has been found, however, that at concentrations above about 40 uM, the gain in variant plants is usually offset by a reduction in the total number of plants obtained. It is particularly preferred, therefore, to employ 6-BA in a concentration range of about 10 uM to about 40 uM. Thus, in accordance with the subject invention, plants regenerated from callus cultured on a basal medium containing high concentrations of 6-BA have shown significantly higher rates of somatic recombination than when the cultures lacked 6-BA. The present invention, therefore, provides a method which increases the probability of recovering desirable recombinants by increasing the total number of recombinants produced.

The optimal exposure to tissue culture conditions will, of course, vary from one plant species to another, however, the determination of time can be ascertained by one skilled in the art based on the criteria that too short exposure will not result in a large number of variants, whilst too long exposure will cause such a large number of deleterious mutations to accumulate that the number of regenerable plantlets will be reduced to an insufficient level for screening purposes.

Following the period of undifferentiated growth, the cultures are induced to regenerate. This regeneration may be initiated by transferring the callus to a medium containing the appropriate hormones and growth regulators to permit regeneration.

It is preferred that certain growth regulators, such as indole acetic acid (IAA) be included along with the 6-BA in the basal tissue culture medium. The growth regulator (IAA), fosters partial regeneration (i.e., shoot formation) thereby limiting the duration of undifferentiated growth and thus avoiding the problems associated with the accumulation of too many undesirable mutations in the plantlet progeny as discussed above. The preferred concentration range of IAA may be employed and such variations are readily within the skill of the art. Generally, however, IAA is employed at concentrations above 1 uM and below 20 uM. Following shoot formation, the entire callus can be transferred to a rooting medium or divided so that only a portion of the callus containing shoots are transferred to the rooting medium. Once the regenerated plantlets have formed roots they may be transferred to pots and grown to mature plants. Seeds can be collected from the regenerated plants, and progeny plants arising therefrom may be screened.

As mentioned above, the subject invention is applicable to any sexually reproducible plant which is capable of being subjected to tissue culture and is recoverable therefrom. To better illustrate the instant invention but not to limit its scope, the following examples are presented.

## EXAMPLE 1

This illustrates the generation, recovery and verification of somatic recombinants from hybrids of Lycopersicon.

Seeds of the MCO strain described above were grown and plants with uniform, normal morphology were identified as donor plants. Young, fully expanded tomato leaves were taken from the donor plants, sterilized by emersion in 7% Clorox for 10 minutes, and rinsed 2-3 times with sterile distilled water. Portions of the leaf tissue, approximately 5 cm X 5 cm, were excised from the leaf with a sterile scalpel and aseptically transferred to a jar containing MS medium (Murashige and Skoog, Physiol. Plant, 14:473-479 (1962)) with the addition of 6-BA at a concentration of 10 uM and IAA at a concentration of 10 uM. A callus mass developed shortly and shoots were regenerated in three to four weeks.

All shoots regenerated from said callus were transferred to a rooting media comprised of one-half strength MS media with 74 uM 3-aminopyridine and 2 uM NAA. Only one to two shoots were regenerated per explant. Plantlets were recovered on rooting medium three to twelve weeks after culture initiation. Between one and three plants were recovered from each explant that regenerated shoots.

Regenerated plants ($R_0$) were transferred to a greenhouse after being placed in soil. The $R_0$ plants were self-fertilized and seed was collected from each regenerated plant to evaluate the next ($R_1$) generation. Seeds for $R_1$ plants were sown in the greenhouse and were transplanted to replicated field plots to evaluate genetic variability.

Nematode resistance was determined by staining for Aps-1 according to the procedure of Medina-Filho, HP and SD Tanksley (In: Evans, D.A. et al.; Eds: "Handbook of Plant Cell Culture, Vol. I, New York, Macmillan Publ. Co. pg. 904) with the following minor modifications. Isozymes were separated on horizontal 12.5% hydrolyzed starch (Sigma) gels run at 5°C with the following buffers: electrodes - 0.3M boric acid, pH 8.6; gel - 0.076M Tris, 0.006M citric acid, pH 8.6. The other traits were determined by scoring for segregation in the selfed progeny.

A total of 61 regenerates, $R_0$ plants, could be unambiguously characterized. The resulting genotypes, grouped into five classes, are summarized in Table 2. The corrotundata trait, coa, which leads to reduced plant height was excluded from the analysis, since the trait is often encountered as an epigenetic phenocopy in tissue culture derived plants. Isozyme analysis showed no recombination involving the Aps-1 locus of any of the regenerates.

## TABLE 2

### SUMMARY OF SOMACLONAL VARIANTS
### RESULTING FROM MITOTIC CROSSING-OVER

| Type | Number of Plants | Genotype | | | Crossover Region* |
|---|---|---|---|---|---|
| A | 42 | Yv/yv | Aps-1(+/1) | C/c | none |
| B | 2 | Yv/yv | Aps-1(+/1) | c/c | III |
| C | 2 | Yv/yv | Aps-1(+/1) | C/C | III |
| D | 4 | Yv/Yv | Aps-1(+/1) | C/c | I & II |
| E | 11 | Yv/Yv | Aps-1(+/1) | C/C | I,II & III |

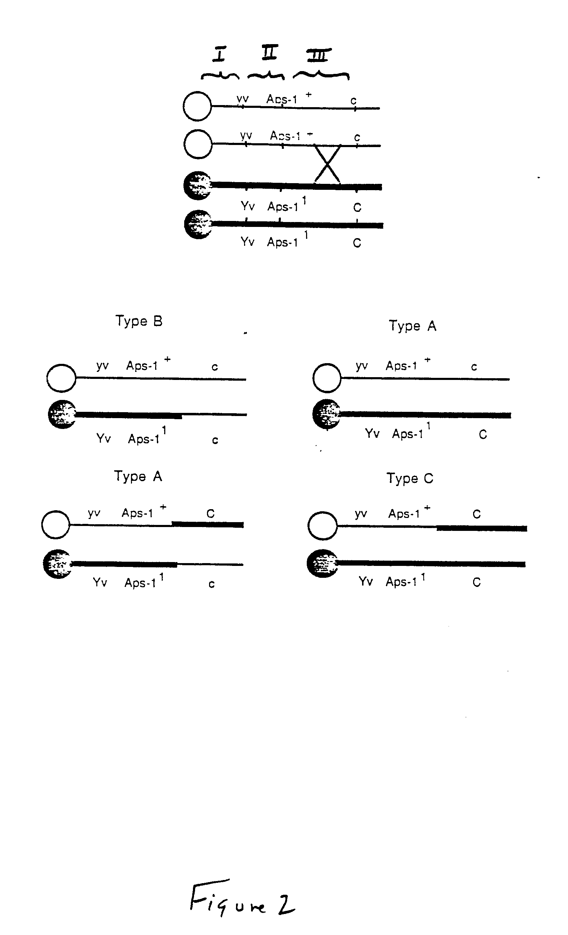

*Based on Figure 2

Based on segregation patterns in the subsequent ($R_1$) generation, 42 regenerates (type A) were unchanged with respect to the three marker loci, yv, Aps-1, and c. Fifteen regenerated plants (D and E types) contained two copies of the wild type allele for yellow virescence. However, no yv/yv combinations were found among the $R_0$ plants. Two regenerates (type B) were homozygous for the recessive potato leaf trait (c/c), while thirteen plants were homozygous for the dominant wild type allele (type C). In addition, 11 regenerates had changes at both the yv and c loci (type E).

Mitotic crossing-over has been suggested as a source of tissue culture derived somaclonal variation. Since organogenesis originates from a single cell, a somatic recombination event will change the genetic make-up of plants which originate from that cell. Therefore, it may be possible to use tissue culture to generate a mitotic crossing-over event to produce a tomato plant homozygous for nematode resistance. Such an approach may break the linkage between the Mi locus and closely linked undesirable traits, e.g. reduced yield and soft fruit, that have apparently been cotransferred from L. peruvianum.

Of the 61 plants examined, none exhibited the Aps-1$^{(1/1)}$ gene combination which would indicate the presence of both nematode resistant alleles. This may reflect a regeneration benefit for the Aps-1 (11+) genotype. A crossover event between Aps-1 and c produced two regenerates (type B) homozygous for the potato leaf allele, c/c. Figure 2 diagrams the mitotic crossing-over event leading to the production of such plants. Loss of the c locus, associated with traits derived from the wild species, may lead to improved agronomic habit for these plants and their progeny.

The frequency of recombination between any two markers on the same arm of the chromosome should reflect the map distance between these two markers. Thus, the ratio of the observed changes were unexpected. Although a single map unit separates yvfrom Aps-1, it was possible to identify 15 mitotic crossing over events between these two traits in 61 plants. In fact, the number of recombinational events observed between yv and Aps-1 is equal to the number between Aps-1 and c, which is 69 map units away. Such a high frequency of recombination was surprising and suggests that the actual chromosome position of these two traits is much farther than a single map unit. The absence of yellow virescent (yv/yv) regenerates may result from in vitro selection in favor of healthier, wild type shoot primordia.

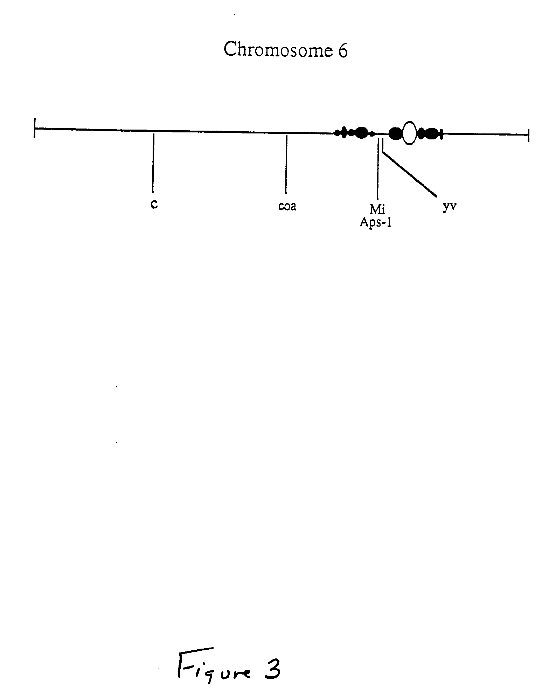

The frequency of meiotic recombination is suppressed in regions adjacent to the centromere. Crossovers near the centromere lead to distorted spindle formation and suppression of recombination in this region would be evolutionarily advantageous. As shown in Figure 3, the yv, Mi, and Aps-1 loci are located near both the centromere and the proximal heterochromatin on chromosome 6L. Thus, calculations of the map distances between these loci near the centromere based on meiotic recombination rates are likely to be abnormally low.

Mitotic crossing-over takes place during chromosome pairing prior to DNA replication, any random pairing of the chromosomes would tend to occur at regions of homology. Since the heterochromatin surrounding the centromere contains mostly repetitive sequences, pairing would be favored in this region and other heterochromatic portions of the chromosomes. Map positions calculated through estimates of mitotic recombination would overestimate distances between markers located near the heterochromatin.

It should be noted that there are two independent processes involved, the mitotic crossing-over event itself and then the regeneration of the resulting recombinants. Thus the probability of recovering a given recombinant plant is a function of both these events.

Discrepancies between map distances generated by meiotic versus mitotic recombination have been reported in yeast and Drosophila. The same phenomenon has now been found in tissue culture induced mitotic recombination of plants. Such an approach has great utility in breaking genetic linkages which could not be broken through meiotic recombination.

## EXAMPLE 2

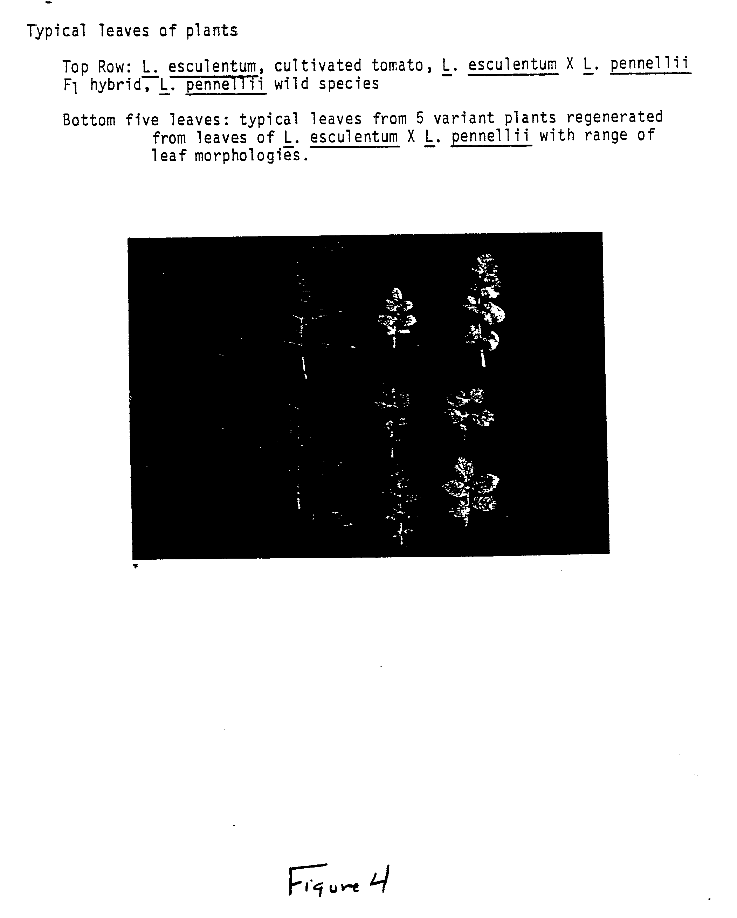

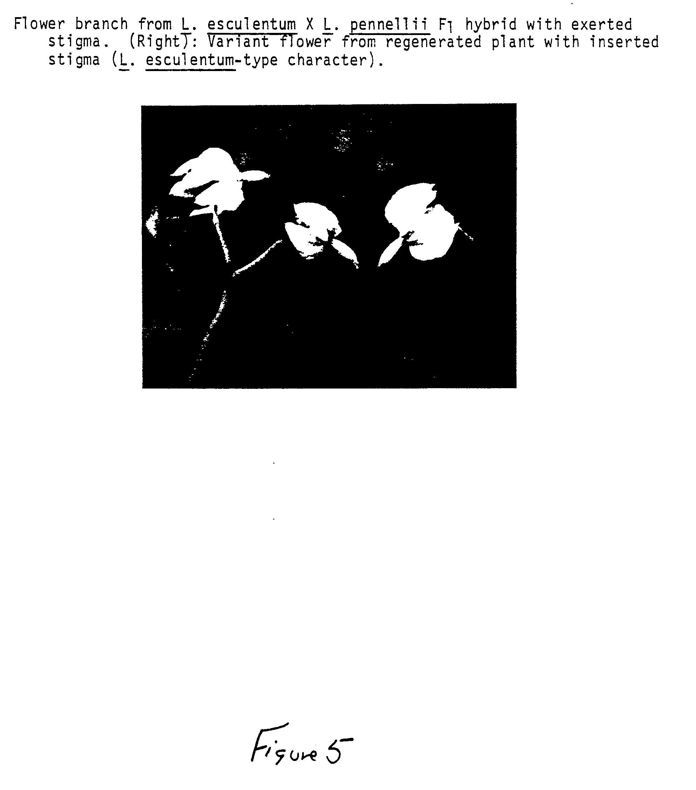

In order to demonstrate additional utility of this invention, an $F_1$ hybrid between a cultivated tomato L. esculentum and a wild species L. pennellii was used as a source of leaf explant tissue and subjected to the tissue culture and regeneration protocol which results in mitotic recombination as described above. As shown in Figures 4 and 5, a variety of somatic recombinants can be recovered.

The hybrid used as a source of explant tissue may be formed by a cross of any two sexually compatible varieties. Hybrids may also be formed by crossing more distantly related varieties, species or even genera by means of embryo rescue, somatic cell hybridization or the like.

By selecting a population of regenerates that had mostly L. esculentum and only some L. pennellii traits it is possible to advance a breeding program by several generations.

## Claims

1. A method for breaking genetic linkage useful for separating desirable traits from undesirable traits in hybrid plants comprising:

a) culturing a callus from a explant of a hybrid plant or from a somatic cell hybrid on a basal medium containing a somatic recombination inducing amount of growth regulator substances and regenerating shoots from said callus;

b) rooting said regenerated shoots on a rooting medium to form regenerated plantlets;

c) growing said plantlets into mature ($R_o$) plants; and

d) recovering those plants displaying a recombinant phenotype.

2. The method according to Claim 1 wherein said explant is selected from the group consisting of cotyledons, hypocotyl, stem, leaf, shoot apex, root, young inflorescenes, flower petals, petioles, ovular tissue and embryos.

3. The method according to Claim 1 wherein said growth regulators are cytokinins and auxins.

4. The method according to Claim 3 wherein said cytokinin is selected from the group consisting of 6-benzyladenine, kinetin, zeatin and 2-isopentyladenine and said auxin is selected from the group consisting of indole acetic acid, indole butyric acid, napthalene acetic acid, and 2,4-dichlorophenoxyacetic acid.

5. The method according to Claim 4 wherein said cytokinins and auxins are 6-benzyladenine and indole acetic acid.

6. The method according to Claim 1 wherein said seed plants are selected from the group consisting of barley, lettuce, maize, oats, onion, pineapple, potato, rape, rice, sorghum, sugarcane, tobacco and tomato.

7. The method according to the Claim 6 wherein said plant is tomato.

8. The method according to Claim 1 wherein after Step c) and before Step d) the mature plants are self-fertilized, seed is collected germinated and $R_1$ plants are grown.

9. The method according to Claim 1 wherein the recovery, step (d), comprises the identification of recombinant plants in the $R_o$ generation.

10. The method according to Claim 1 wherein the recovery, step (d), comprises the identification of recombinant based on lock of segregation in the $R_1$ generation.

11. A method of introgressing useful traits from a wild species into a cultivated species comprising:

a) forming a hybrid between a wild species possessing desirable and undesirable trait and a cultivated variety;

b) culturing a callus from an explant of said hybrid on a basal medium containing a somatic recombination inducing amount of growth regulator substances and regenerating shoots from said callus;

c) rooting said regenerated shoots on a rooting medium to form regenerated plantlets;

d) growing said plantlets into mature ($R_o$) plants; and

e) recovering the plants which display the desired trait in combination with the traits associated with the cultivated variety.

12. The method according to Claim 11 wherein said explant is selected from the group consisting of cotyledons, hypocotyl, stem, leaf, shoot apex, root, young inflorescenes, flower petals, petioles, ovular tissue and embryos.

13. The method according to Claim 11 wherein said growth regulators are cytokinins and auxins.

14. The method according to Claim 13 wherein said cytokinin is selected from the group consisting of 6-benzyladenine, kinetin, zeatin and 2-isopentyladenine and said auxin is selected from the group consisting of indole acetic acid, indole butyric acid, napthalene acetic acid, and 2,4-dichlorophenoxyacetic acid.

15. The method according to Claim 14 wherein said cytokinins and auxins are 6-benzyladenine and indole acetic acid.

16. The method according to Claim 11 wherein said seed plants are selected from the group consisting of barley, lettuce, maize, oats, onion, pineapple, potato, rape, rice, sorghum, sugarcane, tobacco and tomato.

17. The method according to Claim 16 wherein said plant is tomato.

18. The method according to Claim 11 wherein after Step d) and before Step e) the mature plants are self-fertilized, seed is collected, germinated and $R_1$ plants are grown.

UNLINKED

Meiotic diploid
(F.)

| A | B |  | a | c | (Tester) |
|---|---|---|---|---|---|

Testcross progeny

1 4  A B — Parental type
a b

1 4  a b — Parental type
a b

1 4  A b — Recombinant
a b

1 4  a B̄ — Recombinant
a b

LINKED

Meiotic diploid
(F.)

| A | B |  | a | b | (Tester) |
|---|---|---|---|---|---|

Testcross progeny

·1 4  A B — Parental type
a b

~1.4  a b — Parental type
a b

·1 4  A b — Recombinant
a b

·1 4  a B — Recombinant
a b

Figure 1

Figure 2

Chromosome 6

c          coa          Mi          yv
                        Aps-1

Figure 3

Typical leaves of plants

Top Row: L. esculentum, cultivated tomato, L. esculentum X L. pennellii
F₁ hybrid, L. pennellii wild species

Bottom five leaves: typical leaves from 5 variant plants regenerated
from leaves of L. esculentum X L. pennellii with range of
leaf morphologies.

Figure 4

Figure 5

Flower branch from <u>L. esculentum</u> X <u>L. pennellii</u> F₁ hybrid with exerted
stigma. (Right): Variant flower from regenerated plant with inserted
stigma (<u>L. esculentum</u>-type character).

Figure 5